(19) Europäisches Patentamt
European Patent Office
Office européen des brevets

(11) **EP 4 446 330 A1**

(12) **EUROPEAN PATENT APPLICATION**
published in accordance with Art. 153(4) EPC


(43) Date of publication:
**16.10.2024 Bulletin 2024/42**

(21) Application number: **22904757.6**

(22) Date of filing: **09.12.2022**

(51) International Patent Classification (IPC):
***C07H 19/067*** *(2006.01)* ***A61K 31/7068*** *(2006.01)*
***C07K 14/08*** *(2006.01)* ***A61K 45/08*** *(2006.01)*
***A61P 31/16*** *(2006.01)*

(52) Cooperative Patent Classification (CPC):
**A61K 31/00; A61K 31/7068; A61P 31/16;**
**C07H 19/067; C07K 14/08**

(86) International application number:
**PCT/RU2022/000365**

(87) International publication number:
**WO 2023/106964 (15.06.2023 Gazette 2023/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.12.2021 RU 2021136377**



(71) Applicant: **Limited Liability Company "Promomed Rus"**
**Moscow, 129090 (RU)**

(72) Inventors:
- **BELYI, Petr Aleksandrovich**
**Moscow, 127055 (RU)**
- **LOPATUKHIN, Eduard Yurievich**
**Moscow, 125310 (RU)**
- **KOROLEV, Vladimir Lvovich**
**Moscow, 125130 (RU)**
- **ZASLAVSKAYA, Kira Yakovlevna**
**Pushchino, 142290 (RU)**
- **KORLYUKOV, Aleksandr Aleksandrovich**
**Moscow, 119991 (RU)**
- **BUYKIN, Petr Alekseevich**
**Moscow, 111402 (RU)**

(74) Representative: **Zellentin & Partner mbB**
**Patentanwälte**
**Rubensstraße 30**
**67061 Ludwigshafen (DE)**


(54) **NOVEL CRYSTALLINE FORM OF [(2R,3S,4R,5R)-3,4-DIHYDROXY-5-[4-(HYDROXYAMINO)-2-OXOPYRIMIDIN-1-YL]OXOLAN-2-YL]METHYL-2-METHYLPROPANOATE**


(57) The invention relates to the field of medicine, pharmacology and the industry of pharmaceutical chemistry, namely, to a new crystal form IV of [(2R,3S,4R,5R)-3,4-dihydroxy-5- [4-(hydroxyamino)-2-oxopyrimidine -1-yl]oxolan-2-yl]methyl-2-methylpropanoate, as well as pharmaceutical substances, pharmaceutical compositions and medicaments comprising the same and exhibiting prophylactic and/or therapeutic antiviral activity.


EP 4 446 330 A1

## Description

The invention relates to the field of medicine, pharmacology and the industry of pharmaceutical chemistry, namely, to new crystal form IV of [(2R,3S,4R,5R)-3,4-dihydroxy-5-[4-(hydroxyamino)-2-oxopyrimidine-1-yl]oxolan-2-yl]methyl-2-methylpropanoate, as well as pharmaceutical substances, pharmaceutical compositions and medicaments comprising the same and exhibiting prophylactic and/or therapeutic antiviral activity.

One of the most urgent problems of current medicine is the control of viral infections. According to WHO, 2 billion people in the world annually suffer from infectious diseases which cause death for 17 million of them. Every day, 50 thousand deaths in the world are caused by infectious diseases, which still remain among the leading causes of mortality and the first cause of premature death [N. I. Briko, A. Ya. Mindlina, and R. V. Polibin, The universality of changes in the manifestations of the epidemic process of anthroponotic infections over the past decades, Journal of Microbiology, 2015, 5, 12-20].

Most viruses are single-stranded RNA genomes whose replication uses the viral RNA-dependent RNA polymerase. Examples of such viruses are influenza virus, coronavirus, picornavirus, arenavirus, flavivirus, bunyavirus, filovirus, phlebovirus, hantavirus, enterovirus, togavirus, calicivirus, respiratory syncytial virus, parainfluenza virus, rhinoviruses, metapneumoviruses, rotavirus or noravirus.

Nucleoside analogue drugs directly target the activity of RNA-dependent RNA polymerase and block the synthesis of the viral RNA chain for a wide range of RNA viruses, including the family of human coronaviruses (M. L. Agostini et al., Small-molecule antiviral beta-d-N(4)-hydroxycytidine inhibits a proofreading-intact coronavirus with a high genetic barrier to resistance, J. Virol., 2019, 93, e01348-19), influenza viruses (different types) (M. Toots et al., Characterization of oral efficacious influenza drug with high resistance barrier in human airway epithelia, Sci. Transl. Med, 2019, 11, eaax5866. doi: 10.1126/scitranslmed.aax5866), SARS-CoV-2, MERS-CoV (F. Kabinger et al., Mechanism of molnupiravir-induced SARS-CoV-2 mutagenesis, Nature Structural & Molecular Biology, 2021, vol. 28, no. 9, pp. 740-746.), Venezuelan equine encephalitis virus (VEEV) (N. Urakova, et al., β-d-N(4)-Hydroxycytidine Is a Potent Anti-alphavirus Compound That Induces a High Level of Mutations in the Viral Genome, J. Virol., 2018, 92; G. R. Painter et al., The prophylactic and therapeutic activity of a broadly active ribonucleoside analog in a murine model of intranasal Venezuelan equine encephalitis virus infection, Antiviral Res., 2019, 171:104597), Ebola virus (O. Reynard et al., Identification of a new ribonucleoside inhibitor of Ebola virus replication, Viruses, 2015, 7, 6233-6240), norovirus (V. P. Costantini et al., Antiviral activity of nucleoside analogues against norovirus, Antivir. Ther., 2012, 17, 981-991), respiratory syncytial viruses (J. J. Yoon et al. Orally effective broad-spectrum ribonucleoside analog inhibitor of influenza And respiratory syncytial viruses. Antimicrob. Agents Chemother., 2018, 62, e00766-18), Chikungunya virus (M. Ehteshami et al., Characterization of beta-d-N(4)-hydroxycytidine as a novel inhibitor of Chikungunya virus, Antimicrob. Agents Chemother., 2017, 61, e02395-16).

The worldwide outbreak of SARS-CoV-2 and associated consequences thereof pose a threat to the public health and economies of many countries. The lack of specific therapy against the new virus and high variability thereof requires the creation of new drugs.

As part of the control of the new infection, scientists developed numerous prophylactic vaccines, as well as nonspecific therapies that alleviate the course of the disease. However, in addition to existing therapies, there is a need in specific drugs that have a direct effect on the virus, leading to alleviation of the symptoms of the disease, accelerated resolution of the disease, blocking the transmission of infection and reducing the risk of developing clinical complications.

One of the promising nucleoside analogues exhibiting antiviral activity is [(2R,3S,4R,5R)-3,4-dihydroxy-5-[4-(hydroxyamino)-2-oxopyrimidin-1-yl]oxolan-2-yl]methyl-2-methylpropanoate, which is characterized by Formula 1.

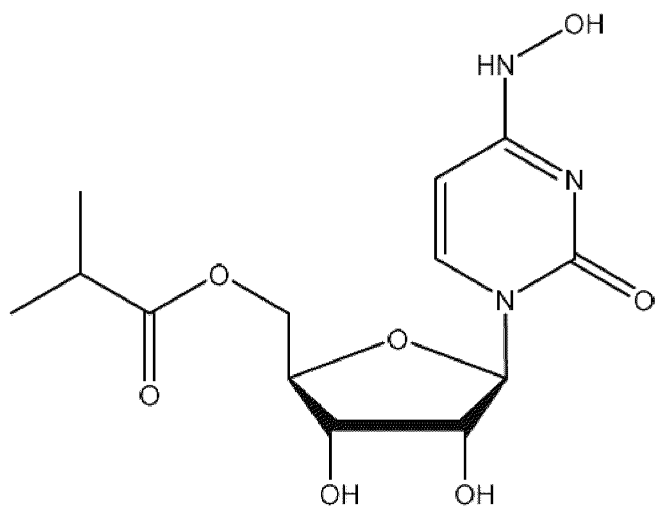

Formula 1

[(2R,3S,4R,5R)-3,4-dihydroxy-5-[4-(hydroxyamino)-2-oxopyrimidin-1-yl]oxolan-2-yl]methyl-2-methylpropanoate is a synthetic nucleoside derivative N4-hydroxycytidine. Its high effectiveness against influenza And coronavirus viruses, including SARS, MERS and SARS-CoV-2, is known from the prior art [Mart Toots et al., Quantitative efficacy paradigms of the influenza clinical drug candidate EIDD-2801 in the ferret model, Translational Research, 2020, vol. 218, pp. 16-28]. The universal mechanism of action of [(2R,3S,4R,5R)-3,4-dihydroxy-5-[4-(hydroxyamino)-2-oxopyrimidin-1-yl]oxolan-2-yl]methyl-2-methylpropanoate is a specific effect on the fundamental enzyme of the viral replication apparatus, which suggests a wide range of antiviral activity of this substance.

When developing drugs, it is important that the substance that is the active agent is in a form with satisfactory physicochemical and technological properties, while exhibiting the own pharmacological activity. This is important regarding the production of pharmaceutical compositions comprising the active agent. The substance that is the active agent, pharmaceutical compositions and medicaments comprising the same should be effectively storable under a wide range of conditions for significant periods of time without exhibiting significant changes in the physicochemical properties of the active agent (for example, chemical composition, density, hygroscopicity, solubility and flowability thereof).

The discovery of new forms of the active agent opens up the possibility of improving the profile of the physicochemical and technological characteristics of a pharmaceutical product (drug). This expands the drug developer's range of active agents when developing a new drug with improved characteristics.

The ability to select a specific crystal form of the same substance among several known forms with different physical and chemical properties allows a person skilled in the art to select the most suitable option for preparing various dosage forms, as well as treatment regimens.

Thus, there is currently a continuing need to develop other, improved forms of antiviral agents.

Active pharmaceutical substances in solid form can exist in a crystalline or amorphous state. Crystalline substances are characterized by the presence of long-range order, and amorphous substances are characterized by its absence. In turn, crystalline substances are characterized by a strict arrangement of atoms in a crystallographic unit cell. The same substance can exist in several crystal forms, which will differ from each other in structure. The structure of a substance directly affects its properties.

Different crystal forms can be characterized and identified using X-ray diffraction analysis, which is based on the ability of X-rays to reflect off the planes formed by atoms in the crystal lattice. Based on X-ray diffraction analysis data, the parameters of a crystallographic unit cell are obtained.

The size of a crystallographic unit cell is determined by three parameters: the length of the cell axes, the relative angles of inclination of the axes to each other, and the volume of the cell. The lengths of the axes of a crystallographic unit cell are determined by the values of a, b and c. The relative angles of inclination of the cell axes are determined by the values of $\alpha$ (alpha), $\beta$ (beta) and $\gamma$ (gamma). The cell volume is denoted as V and is determined by calculating the matrix of scalar products of the sides with each other (Gram matrix). The crystal form of a compound can be described by several crystallographic parameters: the dimensions of the axes of the crystallographic unit cell and the angles therebetween, the volume, and the space group. These parameters are determined experimentally by indicating the diffraction pattern from a single crystal or powder sample. A chemical compound can form more than one type of crystal under different conditions. These different crystal forms are called polymorphs. Moreover, the characteristics of crystallographic unit cells are unique for each of the possible forms.

Another method characterizing a crystal form is qualitative X-ray diffraction analysis whereby an experimental or observed diffraction profile is compared with a model profile representing the pure substance in powder form, both

measured at the same analytical temperature, and the measurements for the test form are characterized as a series of 2θ values and intensities.

The method of powder X-ray diffractometry (X-ray phase analysis) allows identifying numerous forms of a solid compound and structures thereof, including crystalline, amorphous and combined forms (mixtures). Structural studies allow finding a relationship between the structure and properties of solids. Determining the relationship between the structure and properties of a substance allows providing the reasonable control over the technological process of creating pharmaceutical substances and medicaments, to reveal the reasons for changes in these properties under the influence of one or another factor, and makes it possible to control more consciously the technological process of creating pharmaceutical substances, as well as drugs comprising the same, and to provide the desirable changes thereof.

The method of X-ray phase analysis is based on the acquisition and subsequent analysis of the pattern resulting from the diffraction of X-rays on the electron density of the atoms of the irradiated solid sample. X-rays are electromagnetic waves with a length of approximately 10.0 to 0.1 Å. The distances between atoms in the space of the crystal lattice of solids vary from units to hundreds of angstroms, which leads to the phenomenon of diffraction.

The diffracted X-ray beam can be considered as the result of multiple interference from the properties of the crystal lattice planes. A lattice is a collection of an infinite number of parallel atomic planes located at equal distances from each other. Reflection of rays will occur from all atomic planes (their series), since the X-ray beam, unlike optical radiation, penetrates deep into the crystal.

A diffraction pattern (profile) means distribution of diffraction intensity depending on its angle. The diffraction pattern is a graph of the intensity of the diffracted beam versus the 2θ value (or interplanar spacing d) and can be considered as "fingerprints of a given crystalline substance", since each crystal form of a certain substance always gives the same set of diffraction peaks, which are unique only to this crystal form (polymorphic modification of the substance).

In this case, the diffraction pattern of a mixture of two or more individual forms of certain substances will be a superposition of their diffraction patterns, that is, a combination of diffraction patterns of individual substances on one diffraction pattern.

Diffraction patterns of crystal forms of substances are presented as separate, sharply defined narrow lines (peaks), while diffraction patterns of amorphous compounds are presented as a broad maximum (halo).

Despite the variety of solid forms of pharmaceutical substances (polymorphs, amorphous forms, solvates, in particular, hydrates, salts, isomers, co-crystals, etc.), diffraction patterns are unique for each form of a particular substance.

Various forms of the same substance may exhibit different properties, such as solubility, hygroscopicity, stability, particle size and shape, density, bioavailability, flowability, compressibility, electrification, etc. These properties are carefully studied and taken into account when preparing medicaments.

Thus, when developing drugs, it is necessary to determine and take into account the features of polymorphic modifications of active agents. in particular, they should be well dissoluble and storable for long periods of time without showing a significant change in physicochemical and pharmacological properties.

The preparation and structure of two crystal forms of [(2R,3S,4R,5R)-3,4-dihydroxy-5-[4-(hydroxyamino)-2-oxopyrimidin-1-yl]oxolan-2-yl]methyl 2-methylpropanoate are known from the prior art (CN112778387, CN113307834).

As the closest prior art, we considered crystal forms I and II [(2R,3S,4R,5R)-3,4-dihydroxy-5-[4-(hydroxyamino)-2-oxopyrimidin-1-yl]oxolan-2-yl]methyl-2-methylpropanoate, which exhibit antiviral activity and are disclosed in CN113307834. Crystal form III of [(2R,3S,4R,5R)-3,4-dihydroxy-5-[4-(hydroxyamino)-2-oxopyrimidin-1-yl]oxolan-2-yl]methyl-2-methylpropanoate, as claimed in the same document, was not used as the closest analogue since it was not obtained as such and the method of production was not disclosed for it.

Thus, the object of the present invention was to develop a shelf-stable new crystal form IV of [(2R,3S,4R,5R)-3,4-dihydroxy-5-[4-(hydroxyamino)-2-oxopyrimidin-1-yl]oxolane-2-yl]methyl-2-methylpropanoate with improved physicochemical and technological characteristics.

As a result of studies, we prepared a new crystal form IV of [(2R,3S,4R,5R)-3,4-dihydroxy-5-[4-(hydroxyamino)-2-oxopyrimidin-1-yl]oxolan-2-yl]methyl-2-methylpropanoate. Our studies of the properties of crystal form IV of [(2R,3S,4R,5R)-3,4-dihydroxy-5-[4-(hydroxyamino)-2-oxopyrimidin-1-yl]oxolan-2-yl]methyl-2-methylpropanoate according to the present invention have unexpectedly demonstrated, in addition to improving the stability of the new crystal form IV of [(2R,3S,4R,5R)-3,4-dihydroxy-5-[4-(hydroxyamino)-2-oxopyrimidin-1-yl]oxolan-2-yl]methyl-2-methylpropanoate, an improvement of other characteristics, namely, improvement of solubility and reduction of hygroscopicity.

Thus, the technical effects of the present invention are:

- improving the antiviral activity of the new crystal form IV of [(2R,3S,4R,5R)-3,4-dihydroxy-5-[4-(hydroxyamino)-2-oxopyrimidin-1-yl]oxolan-2-yl]methyl-2-methylpropanoate;
- improving the physicochemical and technological properties of the new substance, which is essentially crystal form IV of [(2R,3S,4R,5R)-3,4-dihydroxy-5-[4-(hydroxyamino)-2-oxopyrimidine-1-yl]oxolan-2-yl]methyl-2-methylpropanoate according to the present invention, pharmaceutical compositions and medicaments comprising the same;

in particular, improving storability (in particular, in various climatic zones) and photostability; improving solubility and reducing hygroscopicity.

- expanding the range of antiviral agents with new physicochemical and technological properties without reducing the activity thereof.

The following are definitions of terms that are used in the present specification.

Unless otherwise specified, all technical and technical terms used in the context hereof have their common meaning in the art.

In the context of the present invention, the term "pharmaceutically acceptable excipient" characterizes substances of inorganic or organic origin used when manufacturing medicaments to impart the necessary physical and chemical properties thereto.

In the context of the present invention, the term "pharmaceutical composition" means a composition comprising a therapeutically active agent and at least one of pharmaceutically acceptable and pharmacologically compatible excipients, such as, but not limited to, excipients, solubilizers, solvents, co-solvents, antioxidants, buffering agents, cryoprotectants, diluents, preservatives, stabilizers, humectants, emulsifiers, lubricants, glidants, suspending agents, thickeners, binding agents, sweeteners, fragrances, effervescent agents, flavorings, antibacterial agents, fungicides, sustained release regulators, isotonic agents, pH regulating agents, which selection and ratio depend on the nature, purpose and dosage of the therapeutically active agent.

Non-limiting examples of suspending agents comprise ethoxylated alcohol, isostearyl alcohol, polyoxyethylene, sorbitol and sorbitol ether, and mixtures thereof.

Stabilizers that can be used comprise, but are not limited to, mannitol, gelatose, microcrystalline cellulose, sodium carboxymethylcellulose, polyvinylpyrrolidone, colloidal silica, polysorbate-80, croscarmellose sodium, sodium lauryl sulfate.

Mannitol, ascorbic acid, tocopherol, β-carotene, and lycopene can be used as antioxidants in a pharmaceutical composition.

Starch, glucose, sucrose, lactose, magnesium oxide, sodium chloride, sodium bicarbonate, kaolin, cellulose derivatives, calcium carbonate, dextrin, sorbitol, croscarmellose sodium, hydroxypropyl methylcellulose, magnesium stearate can be used as fillers in the pharmaceutical composition.

Protection of the pharmaceutical composition from the action of microorganisms can be achieved using a variety of antibacterial and antifungal agents, for example, sorbic acid, parabens (methylparaben, propylparaben, ethylparaben, butylparaben, isobutylparaben, isopropylparaben, benzylparaben, heptylparaben and mixtures thereof), chlorobutanol and the like compounds.

As isotonic agents, the pharmaceutical composition may include, but is not limited to, sugars, sodium chloride, sodium bicarbonate, etc. Prolonged action of the pharmaceutical composition can be achieved, without limitation, by using agents that slow down the absorption of the active agent (for example, hydrophilic and hydrophobic polymer release retardants).

Non-limiting (illustrative) examples of suitable fillers comprise lactose, various types of starch, mannitol, microcrystalline cellulose, calcium carbonate and phosphate, etc.

Water, organic esters suitable for parenteral forms, ethanol, polyalcohols, and mixtures thereof, without limitation can be used as solvents and diluents. Examples of lubricants comprise, but are not limited to, magnesium or calcium stearate, talc, sodium lauryl sulfate, sodium stearyl fumarate. Silica, talc, kaolin, bentonites, etc. can act as glidants. Various organic and inorganic acids or alkalis can be used to adjust the pH, such as, but not limited to, hydrochloric acid, malic, ascorbic, citric, acetic, succinic, tartaric, fumaric, lactic, aspartic, glutaric, glutamic, sorbic acid, sodium hydroxide, etc. Examples of dispersing agents and distributing agents are starch, alginic acid and salts thereof, and silicates.

The pharmaceutical composition can be administered to animals and humans orally, parenterally (intradermally, subcutaneously, intramuscularly, intravenously, intraarterially, in the cavity), sublingually, locally, including but not limited to, ophthalmic, nasal administration, etc., rectally in a standard form of administration, in the form of a mixture with traditional pharmaceutical carriers. Suitable unit dosage forms comprise (without limitation) oral forms: tablets, capsules, pellets, granules, powders, solutions, oral and nasal spray solutions, syrups, suspensions, etc., oral: solutions, suspensions, emulsions, concentrates for preparation of injection and infusion dosage forms, aerosols and powders for inhalation administration, pulmonary powders (powders for spraying with an inhaler directly into the lungs, powders and lyophilisates for the preparation of injection and infusion dosage forms; rectal: suppositories, capsules, etc., and eye drops.

In the context of the present invention, the term "pharmaceutically acceptable" means that the substance or composition to which this term is applied should be chemically and/or toxicologically compatible with the other ingredients included in the preparation, and safe for those treated with this substance or composition.

In the context of the present invention, the term "pharmaceutically acceptable salt" means relatively safe and non-toxic organic and inorganic salts of the acids and bases claimed herein.

In the context of the present invention, the term "medicament" characterizes substances or combinations thereof that come into contact with the human or animal body, penetrate the organs and tissues of the human or animal body, and are used for the prevention or treatment of diseases. Medicaments comprise drugs as finished dosage forms intended for administration into the animal or human body in various ways, for example, but not limited to, orally, sublingually, topically, rectally, parenterally.

In the context of the present invention, the term "solid form" means pharmaceutical substances, pharmaceutical compositions and drugs of a solid, dense or viscous consistency, while the particles actively interact with each other, allowing the substance to maintain the own size and shape.

In the context of the present invention, the term "crystallographic unit cell" means a part of a crystal lattice, the parallel transfers of which in three dimensions (translations) make it possible to construct the entire crystal lattice.

In the context of the present invention, the term "pharmaceutical substance" or "substance" means a technological product that is essentially crystal form IV of [(2R,3S,4R,5R)-3,4-dihydroxy-5-[4-(hydroxyamino)-2-oxopyrimidin-1-yl]oxolan-2-yl]methyl-2-methylpropanoate or a mixture of various solid forms (including crystalline and amorphous) thereof, where for the most part the crystal form according to the present invention is present, synthetic or otherwise (biological, biochemical, mineral, plant, animal, microbial and other) origin, intended for the production of pharmaceutical compositions and medicaments, which, during the production of a medicament (drug), becomes the active agent of this medicament and determines the effectiveness and physicochemical properties thereof. Such substances are intended to exhibit pharmacological activity or other direct effect in the diagnosis, treatment, alleviation of symptoms, or prevention of disease.

In the context of the present invention, the term "comprising" and "comprise" means that the specified pharmaceutical substances, pharmaceutical compositions and medicaments comprise the listed components, but do not prevent the inclusion of other components.

In the context of the present invention, the term "therapeutically effective amount" means an amount of a substance (as well as a combination of substances) that, when administered to a subject for the treatment or prevention of a disease, or at least one of the clinical symptoms of a disease or disorder, is sufficient to effect such treatment (prevention). for a disease, disorder or symptom. An "effective amount" is determined depending on the disease, disorder and/or symptoms of the disease or disorder, the severity of the disease, disorder and/or symptoms of the disease or disorder, the clinical presentation and general health of the patient, and the age and/or weight of the subject receiving such treatment (prevention) is necessary.

As used herein, the term "dosage" means the content of one or more active agents in quantitative terms per unit dose, or unit volume, or unit mass according to the drug, or for some types of drugs, the amount of active agent released from the drug per unit of time.

As used herein, the term "powder" means a state of matter in which a solid substance or substances included in the composition thereof are in a free disperse system without a dispersion medium with a dispersion phase in the form of solid particles of various shapes.

In the context of the present invention, the term "crystal form" means a spatially homogeneous, equilibrium state of a substance characterized by a certain elemental composition and structure. The crystalline phase can be represented by various crystal forms that have the same chemical composition, but are characterized by different structure and crystal size.

In the context of the present invention, the term "polymorph" means a specific crystal form of one chemical compound that differs from other forms of the same chemical compound in physicochemical properties. Each polymorph is characterized by certain crystallographic unit cell parameters and symmetry group, as well as a diffraction profile. Thus, the polymorphs of a chemical compound of the present invention are different crystal forms of a particular compound having the same chemical formula, but each polymorph may have different physicochemical properties from other polymorphs. In particular, physicochemical properties may differ, such as solubility profile, hygroscopicity, particle shape, stability, bioavailability, electrification, flowability, compressibility, caking, etc.

As used herein, the term "powder X-ray diffraction" means an analytical method that allows qualitatively and quantitatively determining various forms of substances in a mixture thereof based on the analysis of the diffraction pattern created when the sample under study is irradiated with X-rays. Crystalline substances exhibit precise diffraction maxima that appear near specific angles consistent with the interplanar spacing of the crystal lattice. In contrast, amorphous substances do not have long-range order and produce a broadened peak in the form of a halo in the diffraction pattern. Powder X-ray diffraction is the most effective method for identifying various types of solid forms of matter.

The term "2 theta value" or "2$\theta$" means the position of the peak in the diffraction pattern of the sample under study and is the common unit of x-axis in diffraction patterns. An X-ray beam falls on certain planes of crystal lattices at angle $\theta$ and is reflected therefrom. And a diffraction maximum is observed at angle $\theta$ for this type of plane.

The term "number of molecules (other formula units) in a crystallographic unit cell" or "Z" is the minimum set of formula units (atoms or molecules) that describes the chemical composition of a crystal and has a formal charge of zero.

Parameters a, b, c, $\alpha$ (alpha), $\beta$ (beta), $\gamma$ (gamma) are the unit cell parameters characterizing the crystal system

and symmetry group. Selection of axes a, b, c and related angles α (alpha), β (beta), γ (gamma) can be arbitrary, provided that axes a, b, c do not simultaneously lie in the same plane and intersect at one point. However, it is generally accepted, but not mandatory, in crystallographic practice to select axes corresponding to the following arrangement: angle α (alpha) is formed by axes c and b, angle β (beta) is formed by axes a and c, angle γ (gamma) is formed by axes a and b.

The term "syngony" is a division of crystals based on the symmetry of their crystallographic unit cell. Syngony is characterized by the relationships between the cell axes a, b, c and angles α, β, γ. There are seven crystallographic systems: cubic (in particular, $a = b = c$, $\alpha = \beta = \gamma = 90°$), tetragonal (in particular, $a = b \neq c$, $\alpha = \beta = \gamma = 90°$), hexagonal (in particular, $a = b = c$, $\alpha = \beta = 90°$, $\gamma = 120°$), trigonal (in particular, $a = b = c$, $\alpha = \beta = \gamma \neq 90°$), orthorhombic (in particular, $a \neq b \neq c$, $\alpha = \beta = \gamma = 90°$), monoclinic (in particular, $a \neq b \neq c$, $\alpha = \gamma = 90°$, $\beta \neq 90°$), triclinic (in particular, $a \neq b \neq c$, $\alpha \neq \beta \neq \gamma \neq 90°$). Being the largest classification division in the symmetry of crystals, each system comprises several space groups of symmetry.

The term "space group" is a set of crystal lattice symmetry transformations combining this lattice with itself. There are a total of 230 different space symmetry groups. Of these, 32 groups are used in crystallography to describe symmetry, they are also called point symmetry groups.

As used herein, the term "about" means approximately plus or minus ten percent of the stated value.

In the context of the present invention, the term "±" characterizes the quantitative values (volume and parameters of a crystallographic unit cell) depending on the characteristics of the measuring device, sample preparation, and measurement temperature. These values characterize the ranges, where the reproducibility of the results is observed.

In the context of the present invention, the term "a substance which is essentially crystal form IV of [(2R,3S,4R,5R)-3,4-dihydroxy-5-[4-(hydroxyamino)-2-oxopyrimidin-1-yl]oxolan-2-yl]methyl-2-methylpropanoate" means a substance that has less than about 30 wt.% other solid forms of [(2R,3S,4R,5R)-3,4-dihydroxy-5-[4-(hydroxyamino)-2-oxopyrimidin-1-yl]oxolan-2-yl]methyl-2-methylpropanoate (in particular, less than 25 wt.%, less than 20 wt.%, less than 15 wt.%, less than 10 wt.%, less than 5 wt.%, less than 2 wt.% or none at all). At the same time, the total content of impurities in the substance corresponds to regulatory documentation.

In the context of the present invention, the term "other solid forms of [(2R,3S,4R,5R)-3,4-dihydroxy-5-[4-(hydroxyamino)-2-oxopyrimidin-1-yl]oxolan-2-yl]methyl-2-methylpropanoate" means amorphous and/or crystal forms of [(2R,3S,4R,5R)-3,4-dihydroxy-5-[4-(hydroxyamino)-2-oxopyrimidin-1-yl]oxolan-2-yl]methyl-2-methylpropanoate differing in their structure from the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention.

In the context of the present invention, the term "impurities" means any component of a pharmaceutical substance that is not the active agent. The quality indicator of a pharmaceutical substance characterizes the content of impurities in the pharmaceutical substance that entered the substance during the synthesis thereof, both industrial (technological impurities) and related ones, as well as those formed during the degradation during storage thereof, and is expressed as a percentage. The source of impurities can be reagents used in the synthesis of starting substances, catalysts, technological equipment, residues of filter materials, insufficiently purified starting materials used for the synthesis of pharmaceutical substances.

In the context of the present invention, the term "amorphous form" means a solid substance that does not have a distinguishable crystal lattice, and in which there is no unlimited repeating order over long distances in the relative arrangement of molecules in the substance inherent in crystals. In particular, an amorphous form is characterized by a diffuse maximum (halo) and exhibits almost no sharp peaks in the X-ray diffraction pattern, however, with some changes, distinguishable peaks can be detected against the background of a diffuse maximum.

In the context of the present invention, the term "physicochemical properties of substances" means the density, shape, size and nature of the surface of particles, specific surface area of particles, adhesion forces (sticking together on the surface) and cohesion (sticking together of particles inside the body), surface activity, melting point, solubility, stability, reactivity of substances, etc.

In the context of the present invention, the term "technological properties of substances" means the bulk density, degree of compaction, flowability, humidity, fractional composition, dispersity, porosity, compressibility of substances, etc.

The stated problem is solved, and the stated technical effect is achieved by preparing a new crystal form of [(2R,3S,4R,5R)-3,4-dihydroxy-5-[4-(hydroxyamino)-2-oxopyrimidin-1-yl]oxolan-2-yl]methyl-2-methylpropanoate characterized by monoclinic syngony.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by a space group of the crystallographic unit cell selected from the group of $P2_1$, $P2_1/c$, $P2_1/n$.

More preferably, the crystal form of [(2R,3S,4R,5R)-3,4-dihydroxy-5-[4-(hydroxyamino)-2-oxopyrimidin-1-yl]oxolan-2-yl]methyl-2-methylpropanoate is characterized by space group $P2_1/c$ of the crystallographic unit cell.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the number of molecules in the crystallographic unit cell Z = 4.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: 28.2±2.3 Å; 8.7±2.3 Å; 6.4±2.3 Å, wherein each parameter represents the length of one of axes a, b or c.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by the following values of the crystallographic unit cell parameters: 27.6±0.9 Å, 8.7±0.9 Å, 6.5±0.9 Å, wherein each parameter represents the length of one of axes a, b or c.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by the following values of the crystallographic unit cell parameters: 27.1±0.9 Å, 6.4±0.9 Å, 8.8±0.9 Å, wherein each parameter represents the length of one of axes a, b or c.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by the following values of the crystallographic unit cell parameters: 29.6±0.9 Å, 8.7±0.9 Å, 6.5±0.9 Å, wherein each parameter represents the length of one of axes a, b or c

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by the following values of the crystallographic unit cell parameters: 27.0±0.9 Å, 8.7±0.9 Å, 6.5±0.9 Å, wherein each parameter represents the length of one of axes a, b or c.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: 90.0°; 100.5±20.0°; 90.0°, wherein each of the unit cell parameters represents one of angles α, β or γ between the axes.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: 90.0°; 100.8±14.6°; 90.0°, wherein each of the unit cell parameters represents one of angles α, β or γ between the axes.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by the following values of the crystallographic unit cell parameters: 90.0°, 101.6±1.3°, 90.0°, wherein each of the unit cell parameters represents one of angles α, β or γ between the axes.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by the following values of the crystallographic unit cell parameters: 90.0°, 87.5±1.3°, 90.0°, wherein each of the unit cell parameters represents one of angles α, β or γ between the axes.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by the following values of the crystallographic unit cell parameters: 90.0°, 114.0±1.3°, 90.0°, wherein each of the unit cell parameters represents one of angles α, β or γ between the axes.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by the following values of the crystallographic unit cell parameters: 90.0°, 91.9±1.3°, 90.0°, wherein each of the unit cell parameters represents one of angles α, β or γ between the axes.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by a crystallographic unit cell volume equal to 1525.3 $Å^3$ ± 24.0 $Å^3$.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by a crystallographic unit cell volume equal to 1526.2 $Å^3$ ± 22.9 $Å^3$.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by a crystallographic unit cell volume equal to 1530.3 $Å^3$ ± 23.0 $Å^3$.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by a crystallographic unit cell volume equal to 1524.4 $Å^3$ ± 22.9 $Å^3$.

One preferred embodiment of the invention is the crystal form of [(2R,3S,4R,5R)-3,4-dihydroxy-5-[4-(hydroxyam ino)-2-oxopyrim idin-1-yl]oxolan-2-yl]methyl-2-methylpropanoate characterized by a crystallographic unit cell volume

equal to 1524.3 $Å^3 \pm 22.9 Å^3$.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: a = 27.6±0.9 Å, b = 8.7±0.9 Å, c = 6.5±0.9 Å; $\alpha$ = 90.0°, $\beta$ = 101.6±1.3°, $\gamma$ = 90.0°.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: a = 27.6±0.9 Å, b = 8.7±0.9 Å, c = 6.5±0.9 Å; $\alpha$ = 90.0°, $\beta$ = 101.6±1.3°, $\gamma$ = 90.0°; the volume of a crystallographic unit cell is 1526.2 $Å^3 \pm 22.9 Å^3$.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: a = 27.1 ±0.9 Å, b = 6.4±0.9 Å, c = 8.8±0.9 Å; 90.0°, $\beta$ = 87.5±1.3°, $\gamma$ = 90.0°.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: a = 27.1 ±0.9 Å, b = 6.4±0.9 Å, c = 8.8±0.9 Å; 90.0°, $\beta$ = 87.5±1.3°, $\gamma$ = 90.0°; the volume of a crystallographic unit cell is 1530.3 $Å^3 \pm 23.0 Å^3$.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: a = 29.6±0.9 Å, b = 8.7±0.9 Å, c = 6.5±0.9 Å; 90.0°, $\beta$ = 114.0±1.3°, $\gamma$ = 90.0°.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: a = 29.6±0.9 Å, b = 8.7±0.9 Å, c = 6.5±0.9 Å; 90.0°, $\beta$ = 114.0±1.3°, $\gamma$ = 90.0°; the volume of a crystallographic unit cell is 1524.4 $Å^3 \pm 22.9 Å^3$.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: a = 27.0±0.9 Å, b = 8.7±0.9 Å, c = 6.5±0.9 Å; 90.0°, $\beta$ = 91.9±1.3°, $\gamma$ = 90.0°.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the following values of the crystallographic unit cell parameters: a = 27.0±0.9 Å, b = 8.7±0.9 Å, c = 6.5±0.9 Å; 90.0°, $\beta$ = 91.9±1.3°, $\gamma$ = 90.0°; the volume of a crystallographic unit cell is 1524.3 $Å^3 \pm 22.9 Å^3$.

One embodiment of the invention is the crystal form of [(2R,3S,4R,5R)-3,4-dihydroxy-5-[4-(hydroxyamino)-2-oxopyrimidin-1-yl]oxolan-2-yl]methyl-2-methylpropanoate characterized by the peaks in the powder diffraction pattern at the following angles of $2\theta$ (± 0.2°): 8.30°, 10.35°, 11.10°.

More preferably, the crystal form of [(2R,3S,4R,5R)-3,4-dihydroxy-5-[4-(hydroxyamino)-2-oxopyrimidin-1-yl]oxolan-2-yl]methyl-2-methylpropanoate is The present invention has peaks on the powder diffraction pattern at the following angles of $2\theta$ (± 0.2°): 6.15°, 8.30°, 10.35°, 11.10°, 12.90°.

In one embodiment of the invention, a powder diffraction pattern was recorded on a diffractometer using monochromatic molybdenum K radiation. To prepare the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention, it is possible to use the following known prior art solvents, or deuterium derivatives or mixtures thereof: water, ammonia, hydrocarbons, including aromatic ones (pentane, xylene, benzene, toluene, etc.); esters and ethers (ethyl acetate, diethyl ether, tetrahydrofuran, 1,4-dioxane, dimethoxyethane, crown ethers, etc.); alcohols, including polyhydric ones, including phenols (phenol, methanol, ethanol, isopropanol, butanol, ethylene glycol, propylene glycol, diethylene glycol, etc.); ketones (acetone, etc.); mixtures of hydrocarbons (kerosene, gasoline, etc.); nitro compounds (nitromethane, etc.); nitriles (acetonitrile, etc.); amides (dimethylformamide, dimethylacetamide, etc.); cyclic and acyclic thiols; alkyl halides (dichloromethane, chloroform, carbon tetrachloride, etc.); aryl halides (chlorobenzene, etc.); amines (triethylamine, etc.); petroleum ether; carbon disulfide; sulfoxides (dimethyl sulfoxide, etc.); carbonates (propylene carbonate, etc.); acids (formic, acetic, etc.); heterocycles (pyridine, morpholine, thiophene, pyrrole, N-methyl-2-pyrrolidone, imidazole, etc.); oxides (propylene oxide); tert-butyl methyl ether; hexamethylphosphortriamide; siloxanes (hexamethyldisiloxane, etc.), as well as supercritical fluids (carbon dioxide, water, methane, ethane, propane, ethylene, propylene, methanol, ethanol, acetone, nitrogen oxides, sulfur oxide) or mixtures thereof.

The new crystal form of [(2R,3S,4R,5R)-3,4-dihydroxy-5-[4-(hydroxyamino)-2-oxopyrimidin-1-yl]oxolan-2-yl]methyl-2-methylpropa may be administered in the form of prodrugs. The prodrug may comprise a covalently linked carrier that releases the active parent drug when administered to a mammalian subject. Prodrugs can be prepared by modifying functional groups present in compounds such that the modifications are cleaved, either through routine manipulation or in vivo, into the parent compounds. Prodrugs include, for example, compounds in which a hydroxyl group is linked to any group that, when administered to a subject, is cleaved to form a free hydroxyl group. Examples of prodrugs comprise, but are not limited to, acetate, formate and benzoate derivatives of alcohol functional groups in the compounds.

Typical prodrugs form the active metabolite by conversion of the prodrug by hydrolytic enzymes, hydrolysis of

amides, lactams, peptides, carboxylic acid esters, epoxides, or cleavage of inorganic acid esters.

The stated problem is solved, and the stated technical effect is also achieved by creating a pharmaceutical substance [(2R,3S,4R,5R)-3,4-dihydroxy-5-[4-(hydroxyamino)-2-oxopyrimidin-1-yl]oxolan-2-yl]methyl-2-methylpropanoate comprising the above-mentioned new crystal form of [(2R,3S,4R,5R)-3,4-dihydroxy-5-[4-(hydroxyamino)-2-oxopyrimidin-1-yl]oxolan-2-yl]methyl-2-methylpropanoate.

One embodiment of the invention is a pharmaceutical substance in a powder form.

One embodiment of the invention is a pharmaceutical substance comprising the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention, characterized in that it additionally comprises other solid forms of [(2R,3S,4R,5R)-3,4-dihydroxy-5-[4-(hydroxyamino)-2- oxopyrimidin-1-yl]oxolan-2-yl]methyl-2-methylpropanoate.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1-yl]oxolane-2-yl]methyl-2-methylpropanoate in an amount of less than 30.0 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1-yl]oxolane-2-yl]methyl-2-methylpropanoate in an amount of less than 25.0 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1-yl]oxolane-2-yl]methyl-2-methylpropanoate in an amount of less than 20.0 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1-yl]oxolane-2-yl]methyl-2-methylpropanoate in an amount of less than 15.0 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1-yl]oxolane-2-yl]methyl-2-methylpropanoate in an amount of less than 10.0 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1 -yl]oxolane-2-yl]methyl-2-methylpropanoate in an amount of less than 5.0 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1-yl]oxolane-2-yl]methyl-2-methylpropanoate in an amount of less than 3.0 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1-yl]oxolane-2-yl]methyl-2-methylpropanoate in an amount of less than 2.0 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1-yl]oxolane-2-yl]methyl-2-methylpropanoate in an amount of less than 1.5 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1-yl]oxolane-2-yl]methyl-2-methylpropanoate in an amount of less than 1.0 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is characterized in that it comprises other solid forms of [(2R,3S,4R,5R)-3,4-dihydroxy-5-[4-(hydroxyamino)-2-oxopyrimidin-1-yl]oxolane-2-yl]methyl-2-methylpropanoate in an amount of less than 0.5 wt.%.

More preferably, a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention does not contain other solid forms of [(2R,3S,4R,5R)-3,4-dihydroxy-5-[4-(hydroxyamino)-2-oxopyrim idin-1-yl]oxolan-2-yl]methyl-2-methylpropanoate.

One embodiment of the invention is a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention, in which other solid forms of [(2R,3S,4R,5R)-3,4-dihydroxy-5-[4-(hydroxyamino)-2-oxopyrimidin-1-yl]oxolan-2-yl]methyl-2-methylpropanoate are amorphous and/or crystal forms [(2R,3S,4R,5R)-3,4-dihydroxy-5-[4-(hydroxyamino)-2-oxopyrimidine-1-yl]oxolan-2-yl]methyl-2-methylpropanoate, differing in structure from the new crystal form according to the present invention.

One embodiment of the invention is a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention, in which other solid forms of [(2R,3S,4R,5R)-3,4-dihydroxy-5-[4-(hydroxyamino)-2-oxopyrimidin-1-yl]oxolan-2-yl]methyl-2-methylpropanoate are amorphous, crystal forms of [(2R,3S,4R,5R)-3,4-dihydroxy-5-[4-(hydroxyamino)-2-oxopyrimidin-1-yl]oxolan-2-yl]methyl-2-methylpropanoate, different in structure from the new crystal form according to the present invention, or mixtures thereof.

One embodiment of the invention is a pharmaceutical substance comprising the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention, characterized in that it additionally comprises other solid forms of [(2R,3S,4R,5R)-3,4-dihydroxy-5-[4-(hydroxyamino)-2-oxopyrimidine -1-yl]oxolan-2-yl]methyl-2-methylpropanoate differing in structure from the new crystal form according to the present invention, or mixtures thereof, as well as impurities.

One embodiment of the invention is a pharmaceutical substance comprising the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention comprising impurities.

One embodiment of the invention is a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention, characterized in that the total content of impurities in the composition thereof does not exceed 1.0 wt.% by weight of the pharmaceutical substance.

One embodiment of the invention is a pharmaceutical substance comprising the crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention, characterized in that the total content of impurities in the composition thereof is less than 1.0 wt.%, but more than 0.01 wt.% by weight of the pharmaceutical substance.

One embodiment of the invention is a pharmaceutical substance that exhibits prophylactic and/or therapeutic antiviral activity.

One embodiment of the invention is a pharmaceutical substance for the treatment and/or prevention of viral diseases, where the virus is an RNA virus.

One embodiment of the invention is a pharmaceutical substance for the treatment and/or prevention of viral diseases, where the virus is a virus whose genome is encoded by a single-stranded sense (+) strand as well as an antisense (-) strand of RNA and which uses viral RNA-dependent -RNA polymerase for the replication thereof.

One embodiment of the invention is a pharmaceutical substance for the treatment and/or prevention of viral diseases, where the virus is a highly virulent or low virulent virus.

One embodiment of the invention is a pharmaceutical substance for the treatment and/or prevention of viral diseases, where the virus is an influenza virus, coronavirus, picornavirus, arenavirus, flavivirus, bunyavirus, filovirus, phlebovirus, hantavirus, enterovirus, togavirus, calicivirus, respiratory syncytial virus, parainfluenza virus, rhinoviruses, metapneumoviruses, togavirus, rotavirus or noravirus.

One embodiment of the invention is a pharmaceutical substance for the treatment and/or prevention of viral diseases, where the virus is an encephalitis virus, Ebola virus, SARS-CoV, MERS-CoV, SARS-CoV-2 or influenza A, B.

One embodiment of the invention is a pharmaceutical substance for the treatment and/or prevention of viral diseases, where the virus is an influenza A virus, including strains A (H1N1), A (H1N1) pdm09, A (H1N2), A (H3N2), A (H2N2), A (H4N2), A (H7N2), swine flu type A, avian influenza type A, including highly pathogenic strains (including H5N1 and H7N9).

The stated problem is solved, and the stated technical effect is also achieved by preparing a pharmaceutical composition exhibiting prophylactic and/or therapeutic antiviral activity, comprising in an effective amount the pharmaceutical substance of the present invention.

The stated problem is solved, and the stated technical effect is also achieved by preparing a pharmaceutical composition exhibiting prophylactic and/or therapeutic antiviral activity, comprising in an effective amount the crystal form of [(2R,3S,4R,5R)-3,4-dihydroxy-5-[4 -(hydroxyamino)-2-oxopyrimidin-1-yl]oxolan-2-yl]methyl-2-methylpropanoate according to the present invention.

The stated problem is solved, and the stated technical effect is also achieved by preparing a pharmaceutical composition exhibiting prophylactic and/or therapeutic antiviral activity, comprising in an effective amount the crystal form of [(2R,3S,4R,5R)-3,4-dihydroxy-5-[4 -(hydroxyamino)-2-oxopyrimidin-1-yl]oxolan-2-yl]methyl-2-methylpropanoate according to the present invention, and at least one pharmaceutically acceptable excipient.

The stated problem is solved, and the stated technical effect is also achieved by preparing a pharmaceutical composition exhibiting prophylactic and/or therapeutic antiviral activity, comprising in an effective amount the pharmaceutical substance of the present invention and at least one pharmaceutically acceptable excipient.

The stated problem is solved, and the stated technical effect is also achieved by preparing a pharmaceutical composition exhibiting prophylactic and/or therapeutic antiviral activity, comprising in an effective amount a pharmaceutical substance of crystal form of [(2R,3S,4R,5R)-3,4-dihydroxy-5- [4-(hydroxyamino)-2-oxopyrimidin-1-yl]oxolan-2-yl]methyl-2-methylpropanoate according to the present invention, and at least one pharmaceutically acceptable excipient.

The stated problem is solved, and the stated technical effect is also achieved by preparing a pharmaceutical composition exhibiting prophylactic and/or therapeutic antiviral activity, comprising in an effective amount a pharmaceutical substance, which is essentially a crystal form of [(2R,3S,4R,5R)-3.4 -dihydroxy-5-[4-(hydroxyamino)-2-oxopyrimidin-1-yl]oxolan-2-yl]methyl-2-methylpropanoate according to the present invention, and at least one pharmaceutically acceptable excipient.

In some embodiments, the pharmaceutical composition according to the present invention can be prepared using known conventional methods in the pharmaceutical field.

One embodiment of the invention is a pharmaceutical composition in which an amount of the new crystal form of [(2R,3S,4R,5R)-3,4-dihydroxy-5-[4-(hydroxyamino)-2-oxopyrimidin-1-yl]oxolane The-2-yl]methyl-2-methylpropanoate according to the present invention is from 20 to 6000 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 5800 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 5500 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 5200 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 4900 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 4600 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 4300 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 4000 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 3700 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 3400 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 3100 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 2800 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 2500 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 2200 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 1900 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 1600 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 1300 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 1000 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 700 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 400 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention is from 20 to 100 mg.

One embodiment of the invention is a pharmaceutical composition, where the pharmaceutically acceptable

excipients are selected from the group including excipients, stabilizers, solubilizers, solvents, humectants, lubricants, glidants, thickeners, sweeteners, fragrances, flavoring agents, fungicides, sustained delivery regulators, co-solvents, diluents, fillers, emulsifiers, preservatives, antioxidants, buffering agents, cryoprotectants, pH-regulating agents, isotonicity agents or corrective agents.

One embodiment of the invention is a pharmaceutical composition characterized in that it is a solid pharmaceutical composition.

One embodiment of the invention is a pharmaceutical composition for the treatment and/or prevention of viral diseases, where the virus is an RNA virus.

One embodiment of the invention is a pharmaceutical composition for the treatment and/or prevention of viral diseases, where the virus is a virus whose genome is encoded by a single-stranded sense (+) strand as well as an antisense (-) strand of RNA and which uses viral RNA-dependent -RNA polymerase for the replication thereof.

More preferably, the virus of the present invention is an influenza virus, coronavirus, picornavirus, arenavirus, flavivirus, bunyavirus, filovirus, phlebovirus, hantavirus, enterovirus, togavirus, calicivirus, respiratory syncytial virus, parainfluenza virus, rhinoviruses, metapneumoviruses, togavirus, rotavirus or noravirus.

More preferably, the virus of the present invention is a highly virulent or low virulent virus.

More preferably, the virus of the present invention is an encephalitis virus, Ebola virus, SARS-CoV, MERS-CoV, SARS-CoV-2 or influenza A, B.

More preferably, the influenza virus of the present invention is, but is not limited to, influenza A virus, including strains A(H1N1), A(H1N1)pdm09, A(H1N2), A(H3N2), A(H2N2), A(H4N2), A (H7N2), swine influenza type A, avian influenza type A, including highly pathogenic strains (including H5N1 and H7N9).

The pharmaceutical composition, along with the pharmaceutical substance of the present invention, may comprise other pharmaceutical substances, including those with activity, provided that they do not cause undesirable effects.

The stated problem is solved, and the stated technical effect is also achieved by creating a medicament exhibiting prophylactic and/or therapeutic activity, comprising in an effective amount the pharmaceutical composition according to the present invention.

The stated problem is solved, and the stated technical effect is also achieved by creating a medicament exhibiting prophylactic and/or therapeutic activity, comprising in an effective amount the pharmaceutical composition according to the present invention, including the mentioned pharmaceutical substance of an essentially new crystal form of [(2R,3S,4R,5R)-3,4-dihydroxy-5-[4-(hydroxyamino)-2-oxopyrimidin-1-yl]oxolan-2-yl]methyl-2-methylpropanoate.

The stated problem is solved, and the stated technical effect is also achieved by creating a medicament exhibiting prophylactic and/or therapeutic activity, comprising in an effective amount the pharmaceutical composition according to the present invention, including the mentioned pharmaceutical substance of the present invention, and at least one additional pharmaceutically acceptable excipient.

One embodiment of the invention is a medicament in which an amount of the new crystal form of [(2R,3S,4R,5R)-3,4-dihydroxy-5-[4-(hydroxyamino)-2-oxopyrimidin-1-yl]oxolane The-2-yl]methyl-2-methylpropanoate according to the present invention is from 20 to 6000 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 5800 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 5500 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 5200 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 4900 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 4600 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 4300 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 4000 mg.

More preferably, the amount of the new crystal form of [(2R,3S,4R,5R)-3,4-dihydroxy-5-[4-(hydroxyamino)-2-

oxopyrimidin-1-yl]oxolan-2-yl]methyl-2-methylpropanoate according to the present invention in the medicament is from 20 to 3700 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 3400 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 3100 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 2800 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 2500 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 2200 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 1900 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 1600 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 1300 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 1000 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 700 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 400 mg.

More preferably, the amount of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention in the medicament is from 20 to 100 mg.

One embodiment of the invention is a medicament that is a solid medicament.

More preferably, the solid medicament according to the present invention is, but is not limited to, a tablet, capsule, pellet, dragee, granule, sachet, powder, lyophilisate.

More preferably, the solid medicament according to the present invention is, but is not limited to, a dispersible tablet, including an oral dispersible tablet, a lyophilized tablet, a film-coated tablet, a buccal tablet, an effervescent tablet, a sustained/prolonged/modified release tablet.

Lyophilization (lyophilic drying) is the process of removing solvent from frozen material by sublimation (sublimation) of solvent crystals under vacuum conditions, i.e. turning it into steam, bypassing the liquid phase. The drug in the form of a lyophilisate completely retains the own pharmacological activity.

Solvent removal during freeze-drying is carried out mainly by sublimation. Sublimation is the removal of solvent from a frozen object without the formation of a liquid phase; it is carried out under vacuum or, much less often, in an inert gas. The freezing stage is one of the determining stages for preparing a high-quality drug in the form of a lyophilisate.

Auxiliary pharmaceutically acceptable excipients used in freeze-dried drugs according to the present invention include: solvents, solubilizers (EDTA, β-cyclodextrin, etc.), fillers (mannitol, glycine, glucose, sucrose, lactose, milk and etc.), preservatives (benzyl alcohol, ethyl and methyl parahydroxybenzoate, etc.), pH regulators (buffer solutions, sodium hydroxide, hydrochloric acid), stabilizers, cryoprotectors (dextran, gelatin, hydroxyethyl starch, etc.).

In the form of lyophilisates of the present invention, both individual active agents and mixtures thereof with pharmaceutically acceptable excipients can be presented.

One embodiment of the invention is a medicament that is an oral medicament.

More preferably, the oral medicament according to the present invention is, but is not limited to, a tablet, capsule,

pellet, dragee, granule, powder, syrup or solution.

One embodiment of the invention is a medicament that is a liquid medicament.

More preferably, the liquid medicament according to the present invention is, but is not limited to, a solution, a solution concentrate, a syrup, a suspension.

One embodiment of the invention is a medicament that is a parenteral medicament.

Parenteral administration of drugs is a route of administering drugs into the body in which they bypass the gastrointestinal tract, in contrast to the oral route of drug administration. Parenteral medicaments are sterile preparations intended for administration by injection, infusion, inhalation or implantation into the human or animal body. These comprise solutions, emulsions, suspensions, aerosols, powders and tablets for preparing solutions and implantation, powders for inhalation, lyophilized preparations for preparing dosage forms administered parenterally into the body (subcutaneously, intramuscularly, intravenously, intraarterially, into various cavities).

Parenteral routes of administration comprise administration into tissues (intradermal, subcutaneous, intramuscular, intraosseous), into vessels (intravenous, intraarterial, lymphatic vessels), into cavities (pleural, abdominal, cardiac and articular cavities), into the subarachnoid space, as well as inhalation, intranasal and subconjunctival administration.

More preferably, the parenteral medicament is an infusion solution.

More preferably, the parenteral medicament is an injection solution.

One embodiment of the invention is a medicament that is an inhalation medicament.

More preferably, the inhalation medicament according to the present invention is, but is not limited to, an aerosol, a powder or a pulmonary powder.

One embodiment of the invention is a medicament that is a mild medicament.

Soft dosage forms are ointments, liniments, pastes, patches, porridges, suppositories. All of them have a soft consistency, but they belong to different disperse systems, in other words, they are medicaments with a plastic-elastic-viscous medium.

One embodiment of the invention is a drug that is a drug for topical administration.

Local administration of drugs means the application of a drug to the mucous membranes (including ocular, nasal, rectal, vaginal application, application to the gums, oral mucosa, etc.), as well as administering into the external auditory canal.

More preferably, the topical medicament according to the present invention is a drug for ophthalmic, nasal and rectal administration.

One embodiment of the invention is a medicament that is a rectal medicament.

More preferably, the rectal medicament according to the present invention is, but is not limited to, suppositories or capsules.

One embodiment of the invention is a medicament in which the pharmaceutically acceptable excipients are selected from the group including, but not limited to, excipients, stabilizers, solubilizers, solvents, humectants, lubricants, glidants, thickeners, sweeteners, fragrances, flavoring agents, fungicides, extended delivery regulators, co-solvents, diluents, fillers, emulsifiers, preservatives, antioxidants, buffering agents, cryoprotectants, pH-regulating agents, isotonicity agents or corrective agents.

More preferably, the solvent of the present invention is, but is not limited to, saline solution, water injection, pyrogen-free water, distilled water, Ringer's solution, Hartmann's solution or glucose solution.

The stated problem is solved, and the stated technical effect is achieved through the use of the new crystal form of [(2R,3S,4R,5R)-3,4-dihydroxy-5-[4-(hydroxyamino)-2-oxopyrimidin-1-yl]oxolane-2-yl]methyl-2-methylpropanoate according to the present invention, for the production of a pharmaceutical substance for the treatment and/or prevention of viral diseases.

The stated problem is solved, and the stated technical effect is achieved through the use of a pharmaceutical substance of the new crystal form of [(2R,3S,4R,5R)-3,4-dihydroxy-5-[4-(hydroxyam ino)-2-oxopyrim idin-1-yl]oxolan-2-yl]methyl-2-methylpropanoate according to the present invention for the production of a pharmaceutical composition for the treatment and/or prevention of viral diseases.

The stated problem is solved, and the stated technical effect is achieved through the use of the pharmaceutical composition according to the present invention to obtain a medicament for the treatment and/or prevention of viral diseases.

The stated problem is solved, and the stated technical effect is achieved through the use of the new crystal form of [(2R,3S,4R,5R)-3,4-dihydroxy-5-[4-(hydroxyamino)-2-oxopyrimidin-1-yl]oxolane-2-yl]methyl-2-methylpropanoate according to the present invention for the treatment and/or prevention of viral diseases.

The stated problem is solved, and the stated technical effect is achieved through the use of the new crystal form of [(2R,3S,4R,5R)-3,4-dihydroxy-5-[4-(hydroxyamino)-2-oxopyrimidin-1-yl]oxolane-2-yl]methyl-2-methylpropanoate according to the present invention as part of a pharmaceutical substance contained in a pharmaceutical composition for the treatment and/or prevention of viral diseases.

The stated problem is solved, and the stated technical effect is achieved through the use of the pharmaceutical

substance of the present invention as part of a pharmaceutical composition for the treatment and/or prevention of viral diseases.

The stated problem is solved, and the stated technical effect is achieved through the use of a pharmaceutical substance, which is essentially the new crystal form of [(2R,3S,4R,5R)-3,4-dihydroxy-5-[4-(hydroxyamino)-2- oxopyrimidin-1-yl]oxolan-2-yl]methyl-2-methylpropanoate as part of a pharmaceutical composition for the treatment and/or prevention of viral diseases.

The stated problem is solved, and the stated technical effect is achieved through the use of a pharmaceutical composition or medicament according to the present invention for the treatment and/or prevention of viral diseases.

More preferably, the viral infection of the present invention is an encephalitis virus, Ebola virus, SARS-CoV, MERS-CoV, SARS-CoV-2 or influenza A, B.

The following are examples of implementation of the invention, which illustrate the invention, but do not cover all possible embodiments and do not limit the invention.

A person skilled in the art will understand that other particular embodiments of the invention are possible.

## Examples

### Example 1. Method for preparing the new crystal form of [(2R,3S,4R,5R)-3,4-dihydroxy-5-[4-(hydroxyamino)-2-oxopyrimidin-1-yl]oxolan-2-yl]methyl-2-methylpropanoate.

The calculated amount of [(2R,3S,4R,5R)-3,4-dihydroxy-5-[4-(hydroxyamino)-2-oxopyrimidin-1-yl]oxolan-2-yl]methyl-2-methylpropanoate was dissolved in the mixture acetone/tetrahydrofuran solvents in a mass ratio of 1:3 -2:5, respectively, with stirring with stepwise heating: from 20° up to 35° for an hour; from 35° up to 50° for half an hour. Next, the mixture was kept with uniform stirring at 50° under reduced pressure until the solvent mixture was removed in an amount of 50 to 60% of the total volume. After evaporation of part of the solvent mixture, isopropanol was added dropwise. Next, the resulting mixture was kept at room temperature for 8 hours. Crystallization was observed. The crystals were filtered and dried in a stream of nitrogen at 45-60° for 2.5 to 3 hours. A white crystalline powder was obtained.

Identification of the chemical crystal form of [(2R,3S,4R,5R)-3,4-dihydroxy-5-[4-(hydroxyamino)-2-oxopyrimidin-1-yl]oxolan-2-yl]methyl-2-methylpropanoate was carried out NMR methods and elemental analysis.

$^1$H NMR (600 MHz, CDsOD): $\delta$ 6.93 (d, J = 8.1 Hz, 1H), 5.78 (d, J = 4.6 Hz, 1H), 5.64 (d, J = 8.2 Hz, 1H), 4.26 (d, J = 3, 7 Hz, 2H), 4.14 (t, J = 5.0 Hz, 1H), 4.10 (m, 2H), 2.60 (m, 1H), 1.22 (d, J = 7.2 Hz, 6H); $^{13}$C NMR (75 MHz, CDsOD): $\delta$ 178.4, 151.9, 146.4, 132.1, 99.5, 90.8, 82.7, 74.6, 71.7, 65.3, 35.8, 27.5, 19.7, 19.5.

Elemental analysis of $C_{13}H_{19}N_3O_7$: calculated C 47.42%, H 5.82%, N 12.76%, O 34.01 %; found C 47.54%, H 5.90%, N 12.66%, O 34.08%.

Characteristics of the new crystal form of [(2R,3S,4R,5R)-3,4-dihydroxy-5-[4-(hydroxyamino)-2-oxopyrimidin-1-yl]oxolan-2-yl]methyl-2-methylpropanoate prepared by the method described above was also determined by X-ray diffraction analysis. The X-ray diffraction intensity was measured using a diffractometer using monochromatic molybdenum K$\alpha$ radiation equipped with a position-sensitive detector.

X-ray diffraction pattern of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention showed characteristic individual, sharply defined narrow lines (peaks) (Fig. 1).

Table 1 presents the values of the crystallographic unit cell parameters of the obtained crystal form of [(2R,3S,4R,5R)-3,4-dihydroxy-5-[4-(hydroxyamino)-2-oxopyrimidin-1-yl]oxolan-2-yl]methyl-2-methylpropanoate (diffraction pattern was indicated at $\beta$ setting).

Table 1. Crystallographic values of the parameters of the new crystal form of [(2R,3S,4R,5R)-3,4-dihydroxy-5-[4-(hydroxyamino)-2-oxopyrimidin-1-yl]oxolan-2-yl]methyl-2-methylpropanoate.

| Characteristics of the new crystal form of [(2R,3S,4R,5R)-3,4-dihydroxy-5-[4-(hydroxyamino)-2-oxopyrimidin-1-yl] oxolan-2-yl]methyl-2-methylpropanoate | |
|---|---|
| Crystallographic unit cell parameters | |
| Type of system | Monoclinic |
| Z | 4 |

(continued)

| Crystallographic cell parameters | |
|---|---|
| Axis length a, b or c, Å | 28.2±2.3* |
| | 8.7±2.3* |
| | 6.4±2.3* |
| Angle between axes β, α or γ, ° | 100.5±20.0** |
| | 90.0** |
| | 90.0** |
| V, $Å^3$ | 1525.3 ± 24.0 |
| - where each parameter represents the length of one of axes a, b or c.<br>**- where each parameter represents one of the angles between the axes α, β or γ. | |

Micrographs of the test sample were also obtained with a 200-fold magnification (Fig. 2).

Furthermore, the physicochemical and technological properties of the new crystal form of [(2R,3S,4R,5R)-3,4-dihydroxy-5-[4-(hydroxyamino)-2-oxopyrimidin-1-yl]oxolan-2-yl]methyl-2-methylpropanoate were studied. In addition to improving the stability of the new crystal form of [(2R,3S,4R,5R)-3,4-dihydroxy-5-[4-(hydroxyamino)-2-oxopyrimidin-1-yl]oxolan-2-yl]methyl-2-methylpropanoate, numerous experiments unexpectedly showed improvements in other characteristics, namely, higher solubility and lower hygroscopicity.

**Example 2. Preparation of dosage forms of the new crystal form of [(2R,3S,4R,5R)-3,4-dihydroxy-5-[4-(hydroxyamino)-2-oxopyrimidin-1-yl]oxolan-2-yl]methyl-2-methylpropanoate.**

Solution

The claimed solution was prepared as follows: water for injection was placed in an apparatus for preparing solutions equipped with a mixing device and a thermostatic jacket and heated with continuous stirring to a temperature of 50-65°C, then the calculated amount of mannitol was added and stirred until completely dissolved. The calculated amount of the new crystal form of [(2R,3S,4R,5R)-3,4-dihydroxy-5-[4-(hydroxyamino)-2-oxopyrimidin-1-yl]oxolan-2-yl]methyl was added to the resulting solution-2-methylpropanoate according to the present invention and stirred at a temperature of 60-70°C until a clear solution was obtained. Next, the solution was diluted to a volume of 1000 ml with water for injection and filtered through a 0.22 μm membrane filter.

Capsules

To obtain a solid medicament in the form of capsules comprising the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention as an active agent, the crystal form of [(2R,3S,4R,5R)-3,4-dihydroxy-5-[4-(hydroxyamino)-2-oxopyrimidin-1-yl]oxolan-2-yl]methyl-2-methylpropanoate, lactose and corn starch. The resulting mixture was stirred in a high shear mixer and moistened with a methylcellulose solution. The humidification process was continued until the humidifier was completely added.

The resulting pharmaceutical composition was granulated, followed by drying of the granules in a fluidized bed drier. Next, the granules were calibrated through a sieve with a mesh size of 1 mm.

To prepare the finished pharmaceutical composition for encapsulation, the resulting granulate was stirred with magnesium stearate in a gravimetric mixer. The resulting pharmaceutical composition was encapsulated using a capsule filling machine. Next, the capsules were subjected to dust removal and polishing.

**Example 3. Study of the therapeutic effect of the new crystal form of [(2R,3S,4R,5R)-3,4-dihydroxy-5-[4-(hydroxyamino)-2-oxopyrimidin-1-yl]oxolan-2-yl]methyl-2-methylpropanoate against influenza virus.**

The antiviral properties of the new crystal form of [(2R,3S,4R,5R)-3,4-dihydroxy-5-[4-(hydroxyamino)-2-oxopyrimidin-1-yl]oxolan-2-yl]methyl-2-methylpropanoate in relation to influenza viruses were studied in a model of experimental lethal influenza infection in mice caused by influenza A viruses.

Modeling of viral infection in mice was carried out by intranasal administration of units of highly pathogenic

avian influenza H1N1 virus adapted to mice. The experiment was carried out on 40 mature mice. The animals were kept under standard vivarium conditions and given sterile rodent food and sterile water.

The preparations of the new crystal form of [(2R,3S,4R,5R)-3,4-dihydroxy-5-[4-(hydroxyamino)-2-oxopyrimidin-1-yl]oxolan-2-yl]methyl-2-methylpropanoate were administered orally using a gastric tube in dosages of 400 mg, 800 mg, 1000 mg (all doses are given in terms of per person) 2 times a day for 5 days (groups 1-3, respectively). The antiviral activity of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention, was taken into account by the reduction in mortality of mice in test samples (groups 1-3) compared to the control (group 4, animals of which received a suspension of microcrystalline cellulose as a placebo).

The animals were observed for 8 days. During the experiment, integral indicators (changes in the weight of animals) were assessed daily and clinical symptoms (fever, mucous discharge, activity level, breathing) were assessed, and the mortality of animals in the control and experimental groups was also recorded.

Based on the obtained mortality rates in each group, the percentage of mortality (C) was calculated using the formula:

* C = M:N, where M is the number of animals that died in 8 days, N is the total number of infected animals in the group.

The results of study are presented in Table 2.

Table 2. Results of studying the therapeutic effect of the new crystal form of [(2R,3S,4R,5R)-3,4-dihydroxy-5-[4-(hydroxyamino)-2-oxopyrimidin-1-yl]oxolan-2-yl] methyl 2-methylpropanoate

| Groups | Dose, mg/day (calculated per person) | Number of surviving animals after 14 days | Mortality rate (%) |
|---|---|---|---|
| Group 4 (control) | - | 3/10 | 70 |
| Group 1 | 800 | 10/10 | 0* |
| Group 2 | 1600 | 10/10 | 0* |
| Group 3 | 2000 | 10/10 | 0* |
| * - significant difference in mortality rate from the control group ($p < 0.05$) | | | |

The clinical symptoms of influenza disease, including fever, were observed in experimental mice already on the first day after infection. A day after the start of therapy, groups 1-3 showed a decrease in fever and an increase in physical activity; group 4 (control) showed shortness of breath, a decrease in body weight and an increase in body temperature in animals. By the end of the experiment, the mortality rate of animals in the control group was 70%, while in groups 1-3 the mortality rate was zero.

An additional experiment was also carried out to compare the therapeutic activity of the new crystal form of [(2R,3S,4R,5R)-3,4-dihydroxy-5-[4-(hydroxyamino)-2-oxopyrimidin-1-yl]oxolan-2-yl] methyl 2-methylpropanoate according to the present invention (group 1) and crystal forms I, II (groups 2, 3, respectively) (prototypes), obtained according to the described reference methods of CN113307834.

Therapeutic activity was studied in a model of experimental influenza infection in mice caused by influenza A viruses. The indicated crystal forms [(2R,3S,4R,5R)-3,4-dihydroxy-5-[4-(hydroxyamino)-2-oxopyrimidine-1-yl]oxolan-2-yl]methyl-2-methylpropanoate were administered 24 hours after infection orally by gastric tube at a dosage of 800 mg (all doses are expressed per person) 2 times a day for 5 days. Viral titers in the nasal lavage of experimental animals were assessed daily throughout the experiment.

The clinical symptoms of influenza in mice were noted already on the 1st day of the experiment and were manifested by a decrease in the physical activity of animals, an increase in body temperature, and worsening quality of wool. Already on the second day of the experiment (one day after the administration of the drugs), animals in groups 1-3 showed a decrease in the titer of the virus in the nasal wash and a decrease in fever. On the third day of the experiment, in groups 1-3, the trend towards a decrease in the titer of the virus in the nasal wash continued; however, in group 1, the values of the virus titer in the nasal wash of experimental animals were approximately 20% less than in animals in groups 2 and 3. At the same time, in groups 2 and 3, some animals showed a decrease in body weight and shortness of breath, in contrast to animals of group 1, in whom clinical symptoms of the disease were practically not recorded.

Thus, the new crystal form of [(2R,3S,4R,5R)-3,4-dihydroxy-5-[4-(hydroxyamino)-2-oxopyrimidin-1-yl]oxolan-2-yl]methyl-2-methylpropanoate exhibits improved antiviral activity compared to prior art crystal forms.

**Example 4. Determination of the hygroscopicity of the new crystal form of [(2R,3S,4R,5R)-3,4-dihydroxy-5-[4-(hydroxyamino)-2-oxopyrimidin-1-yl]oxolan-2-yl]methyl-2-methylpropanoate.**

Study of the hygroscopicity of the new crystal form of [(2R,3S,4R,5R)-3,4-dihydroxy-5-[4-(hydroxyam ino)-2-oxopyrimidin-1-yl]oxolan-2-yl]methyl-2-methylpropanoate according to the present invention was carried out by exposure to water vapor from the air based on their experimental storage in an atmosphere with increased partial pressure of water vapor. The creation of a fixed partial pressure of water vapor or a fixed relative air humidity was carried out by using the special equipment of climatic chambers. The following storage conditions were set in the climatic chamber: temperature (25±1)°C and relative humidity (80±2)%.

Polymorphs I and II prepared according to the method described in the prior art (CN113307834) were used as comparison samples in the experiment. The samples were subjected to similar tests of exposure to water vapor from the air.

A sample of the analyzed substance in the amount of 200 mg was placed in a preweighed glass bottle ($m_1$) with a height of 15 mm and an outer diameter of 50 mm. The bottle was covered with a lid and weighed ($m_2$).

Then the sample was placed in a climatic chamber, the lid was removed from the beaker and the sample was kept for 24 hours. After the time had elapsed, the beaker was closed with a lid, taken out of the climatic chamber and weighed ($m_3$).

The increase in the weight of the test substance in percent (X) was calculated using the formula:

$$X = [(m_3 - m_2)/(m_2 - m_1)] \cdot 100,$$

where $m_1$ is the weight (g) mass of an empty glass bottle, $m_2$ is the weight (g) of a glass bottle with a test sample before exposure to a humid environment, and $m_3$ is the weight (g) of a glass bottle with a test sample after exposure to a humid environment.

The measurement results are presented in Table 3 below.

Table 3. Results of studying the hygroscopicity of crystal forms of [(2R,3S,4R,5R)-3,4-dihydroxy-5-[4-(hydroxyam ino)-2-oxopyrim idin-1-yl]oxolan-2-yl]methyl-2-methylpropanoate.

| Test samples | X (%) |
|---|---|
| Crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention | 0.5 |
| Polymorph I (prior art) | 4.1 |
| Polymorph II (prior art | 5.2 |

The results obtained clearly demonstrate a large increase in the mass of the prototypes when stored in a climate chamber, which confirms their hygroscopicity, in contrast to the sample of the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention.

Thus, the new crystal form of [(2R,3S,4R,5R)-3,4-dihydroxy-5-[4-(hydroxyamino)-2-oxopyrimidin-1-yl]oxolan-2-yl]methyl-2-methylpropanoate according to the present invention has reduced hygroscopicity compared to prototypes.

The present invention is further illustrated by Fig. 1 and Fig. 2.

Figure 1 shows the diffraction pattern of the new crystal form of [(2R,3S,4R,5R)-3,4-dihydroxy-5-[4-(hydroxyam ino)-2-oxopyrim idin-1-yl]oxolan-2-yl]methyl-2-methylpropanoate according to the present invention, where I means the intensity measured in %.

Figure 2 shows a micrograph of a sample of the new crystal form of [(2R,3S,4R,5R)-3,4-dihydroxy-5-[4-(hydroxyamino)-2-oxopyrimidin-1-yl]oxolan-2-yl] methyl 2-methylpropanoate according to the present invention with a 200-fold magnification.

## Claims

1. A crystal form of [(2R,3S,4R,5R)-3,4-dihydroxy-5-[4-(hydroxyamino)-2-oxopyrimidin-1-yl]oxolan-2-yl]methyl 2-methylpropanoate **characterized by** monoclinic syngony.

2. The crystal form according to claim 1, **characterized by** the number of molecules in the crystallographic unit cell Z equal to 4.

3. The crystal form according to claim 1, **characterized by** the following values of the crystallographic unit cell parameters: 28.2±2.3 Å; 8.7±2.3 Å; 6.4±2.3 Å, wherein each parameter represents the length of one of axes a, b or c.

4. The crystal form according to claim 1, **characterized by** the following values of the crystallographic unit cell parameters: 90.0°; 100.5±20.0°; 90.0°, wherein each of the parameters represents one of angles $\alpha$, $\beta$ or $\gamma$ between the axes.

5. The crystal form according to claim 1, **characterized by** a volume of the crystallographic unit cell equal to 1525.3±24.0 $A^3$

6. The crystal form according to claim 1, **characterized by** the space group of the crystallographic unit cell selected from the group of $P2_1$, $P2_1/c$, $P2_1/n$.

7. The crystal form according to claim 1, **characterized by** the peaks in the powder diffraction pattern at the following angles 2θ (± 0.2°): 8.30°, 10.35°, 11.10°.

8. The crystal form according to claim 1, **characterized by** the peaks in the powder diffraction pattern at the following angles 2θ (± 0.2°): 6.15°, 8.30°, 10.35°, 11.10°, 12.90°.

9. The crystal form according to claims 7 and 8, **characterized by** the powder diffraction pattern recorded using a diffractometer using monochromatic molybdenum K radiation.

10. A pharmaceutical substance comprising a crystal form according to any one of claims 1-9.

11. The pharmaceutical substance according to claim 10, wherein said pharmaceutical substance is in the form of a powder.

12. The pharmaceutical substance according to any one of claims 10-11, **characterized in that** it further comprises other solid forms of [(2R,3S,4R,5R)-3,4-dihydroxy-5-[4-(hydroxyam ino)-2-oxopyrim idin-1-yl]oxolan-2-yl]methyl-2-methylpropanoate.

13. The pharmaceutical substance according to claim 12, wherein the amount of other solid forms of [(2R,3S,4R,5R)-3,4-dihydroxy-5-[4-(hydroxyamino)-2-oxopyrimidin-1-yl]oxolane-2-yl]methyl-2-methylpropanoate is less than 30.0 wt.%, less than 25.0 wt.%, less than 20.0 wt.%, less than 15.0 wt.%; less than 10.0 wt.%, less than 5.0 wt.%, less than 3.0 wt.%, less than 2.0 wt.%, less than 1.5 wt.%, less than 1.0 wt.% or less than 0.5 wt.%.

14. The pharmaceutical substance according to claim 12, wherein other solid forms of [(2R,3S,4R,5R)-3,4-dihydroxy-5-[4-(hydroxyamino)-2-oxopyrimidin-1-yl]oxolan-2-yl]methyl-2-methylpropanoate are amorphous and/or crystal forms of [(2R,3S,4R,5R)-3,4-dihydroxy-5-[4-(hydroxyamino)-2-oxopyrimidin-1-yl]oxolan-2-yl]methyl-2-methylpropanoate, differing in structure from the crystal form according to any one of claims 1-9.

15. The pharmaceutical substance according to claim 10, **characterized in that** the total content of impurities does not exceed 1.0 wt.%.

16. The pharmaceutical substance according to claim 10, **characterized in that** the total impurity content is less than 1.0 wt.%, but more than 0.01 wt.%.

17. A pharmaceutical composition exhibiting prophylactic and/or therapeutic antiviral activity, comprising in an effective amount the crystal form according to any one of claims 1-9, and at least one pharmaceutically acceptable excipient.

18. The pharmaceutical composition according to claim 17, wherein the amount of the crystal form is from 20 to 6000 mg.

19. The pharmaceutical composition according to claim 18, wherein the amount of the crystal form is from 20 to 5800 mg, from 20 to 5500 mg, from 20 to 5200 mg, from 20 to 4900 mg, from 20 to 4600 mg, from 20 to 4300 mg, from 20 to 4000 mg, from 20 to 3700 mg, from 20 to 3400 mg, from 20 to 3100 mg, from 20 to 2800 mg, from 20 to 2500 mg, from 20 to 2200 mg, from 20 to 1900 mg, from 20 to 1600 mg, from 20 to 1300 mg, from 20 to 1000 mg, from

20 to 700 mg, from 20 to 400 mg, or from 20 to 100 mg.

**20.** The pharmaceutical composition according to claim 17, **characterized in that** the pharmaceutically acceptable excipients are selected from the group including excipients, stabilizers, solubilizers, solvents, humectants, lubricants, glidants, thickeners, sweeteners, fragrances, flavors, fungicides, prolonged delivery regulators, co-solvents, diluents, fillers, emulsifiers, preservatives, antioxidants, buffering agents, cryoprotectants, pH-regulating agents, isotonicity agents or corrective agents.

**21.** The pharmaceutical composition according to claim 17, **characterized in that** it is a solid pharmaceutical composition.

**22.** The pharmaceutical composition according to claim 17, wherein the virus is an RNA virus.

**23.** The pharmaceutical composition according to claim 22, wherein the virus is a virus whose genome is encoded by a single-stranded sense (+) strand as well as an antisense (-) strand of RNA and which uses a viral RNA-dependent RNA polymerase for the replication thereof.

**24.** The pharmaceutical composition according to claim 22, wherein the virus is a highly virulent or low virulent virus.

**25.** The pharmaceutical composition according to claim 22, wherein the virus is an influenza virus, coronavirus, picornavirus, arenavirus, flavivirus, bunyavirus, filovirus, phlebovirus, hantavirus, enterovirus, togavirus, calicivirus, respiratory syncytial virus, parainfluenza virus, rhinoviruses, metapneumoviruses, togavirus, rotavirus or noravirus.

**26.** The pharmaceutical composition according to claim 25, wherein the virus is an encephalitis virus, Ebola virus, SARS-CoV, MERS-CoV, SARS-CoV-2 or influenza A, B.

**27.** A medicament exhibiting prophylactic and/or therapeutic antiviral activity, comprising in an effective amount the pharmaceutical composition according to any one of claims 17-28.

**28.** The medicament according to claim 27, **characterized in that** it is a solid medicament.

**29.** The medicament according to claim 28, **characterized in that** it is a tablet, capsule, powder, lyophilisate, sachet, pellet, granule.

**30.** The medicament according to claim 29, **characterized in that** it is a dispersible tablet, a lyophilized tablet, a film-coated tablet.

**31.** The medicament according to claim 27, **characterized in that** it is an oral medicament.

**32.** The medicament according to claim 27, **characterized in that** it is a liquid medicament.

**33.** The medicament according to claim 32, **characterized in that** it is a solution, a concentrate for preparing a solution, a syrup, a suspension.

**34.** The medicament according to claim 27, **characterized in that** it is a parenteral medicament.

**35.** The medicament according to claim 34, **characterized in that** it is an infusion solution.

**36.** The medicament according to claim 34, **characterized in that** it is an injection solution.

**37.** The medicament according to claim 34, **characterized in that** it is an inhaled medicament.

**38.** The medicament according to claim 37, **characterized in that** the inhaled medicament is an aerosol or pulmonary powder.

**39.** The medicament according to claim 27, **characterized in that** it is a mild medicament.

**40.** The medicament according to claim 27, **characterized in that** it is a rectal medicament.

**41.** Use of a pharmaceutical substance according to any one of claims 10-16 as part of a pharmaceutical composition for the treatment and/or prevention of viral diseases.

**42.** Use of a pharmaceutical composition according to any one of claims 17-26 or a medicament according to any one of claims 27-40 for the treatment and/or prevention of viral diseases.

**43.** Use according to claims 41 and 42, wherein the viral infection is an encephalitis virus, Ebola virus, SARS-CoV, MERS-CoV, SARS-CoV-2 or influenza A, B.

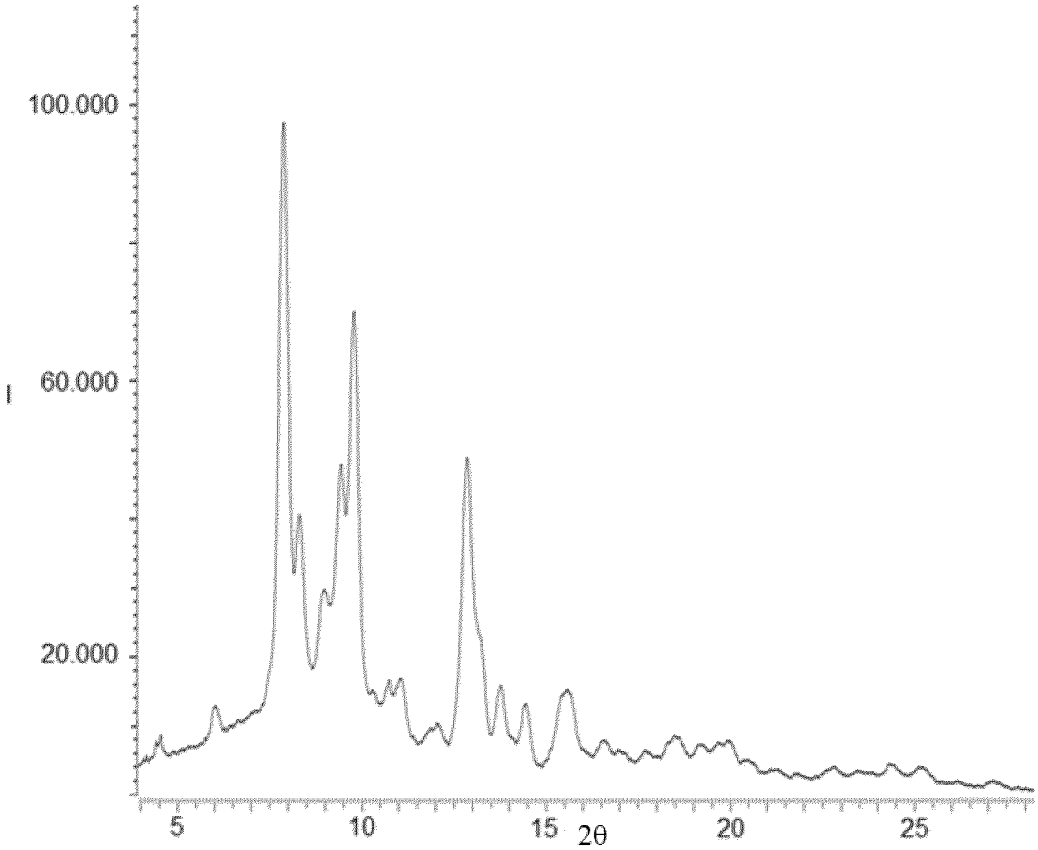


Fig. 1

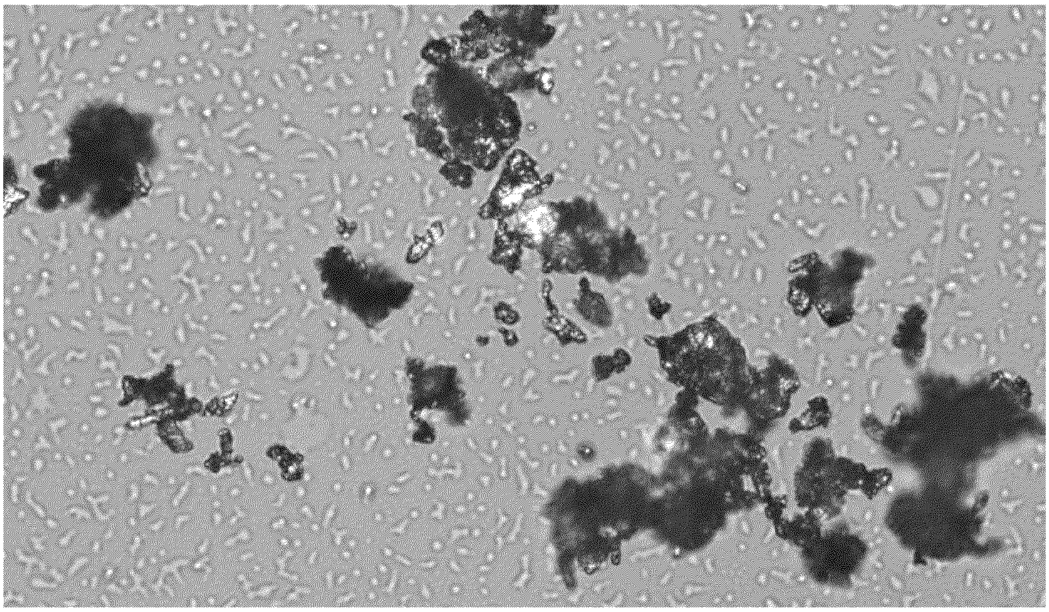

Fig. 2

**INTERNATIONAL SEARCH REPORT**

International application No.
PCT/RU2022/000365

A. CLASSIFICATION OF SUBJECT MATTER
(see the supplemental sheet)

According to International Patent Classification (IPC) or to both national classification and IPC

B. FIELDS SEARCHED

Minimum documentation searched (classification system followed by classification symbols)
C07H 19/06, 19/067, A61K 31/7068, 45/08, A61P 31/14, 31/16, C3OB 29/54, 29/60, C07K 14/08

Documentation searched other than minimum documentation to the extent that such documents are included in the fields searched

Electronic data base consulted during the international search (name of data base and, where practicable, search terms used)
PatSearch (RUPTO Internal), USPTO, PAJ, Espacenet

C. DOCUMENTS CONSIDERED TO BE RELEVANT

| Category* | Citation of document, with indication, where appropriate, of the relevant passages | Relevant to claim No. |
|---|---|---|
| X<br>Y | CN 113307834 A (ANHUI BIOCHEM UNITED PHARMACEUTICAL CO., LTD.) 27.08.2021, example, the claims | 1-6, 10-43<br>7-9 |
| X<br>Y | CN 112778387 A (HANGZHOU CHEMINSPIRE TECHNOLOGIES CO., LTD.) 11.05.2021, example, the claims | 1-6, 10-43<br>7-9 |
| Y | CAIRA Mino R. Crystalline polymorphism of organic compounds. Topics in Cussernt Chemistry. Springer Verlag Berlin Heidelberg, 1998, V.198, p.163-208, doi: 10.1007/3-540-69178-2_5, p. 164-166 | 7-9 |
| Y | SHERRY L. Morissette et al. High-through put crystallization: polymorphs, salts, co-crystals and solvates of pharmaceutical solids. Advanced drug Delivery Reviews, 2004, v.56, pp.275-300, section 1 | 7-9 |
| Y | VARIANKAVAL Narayan et al. From form to function: Crystallization of active pharmaceutical ingredients. AlChE Journal, 2008, vol. 54 (7), pp.1682-1688, doi: 10.1002/aic.11555, page 1682, “Crystal Form” | 7-9 |

[X] Further documents are listed in the continuation of Box C.  [ ] See patent family annex.

* Special categories of cited documents:
“A” document defining the general state of the art which is not considered to be of particular relevance
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| 10 March 2023 (10.03.2023) | 06 April 2023 (06.04.2023) |
| Name and mailing address of the ISA/<br>RU<br><br>Facsimile No. | Authorized officer<br><br>Telephone No. |

Form PCT/ISA/210 (second sheet) (April 2005)

**INTERNATIONAL SEARCH REPORT**

International application No.
PCT/RU2022/000365

C (Continuation). DOCUMENTS CONSIDERED TO BE RELEVANT

| Category* | Citation of document, with indication, where appropriate, of the relevant passages | Relevant to claim No. |
|---|---|---|
| P, X | WO 2022/047229 A1 (EMORY UNIVERSITY et al.) 03.03.2022, claims 1, 15-20 | 1-43 |
| P, X | WO 2022/123334 A1 (OPTIMUS DRUGS PVT LTD) 16.06.2022, claims | 1-6, 10-11, 15-17,20-43 |

Form PCT/ISA/210 (continuation of second sheet) (April 2005)

INTERNATIONAL SEARCH REPORT

International application No.
PCT/RU2022/000365

A. CLASSIFICATION OF SUBJECT MATTER

***C07H 19/067*** (2006.01)
***A61K 31/7068*** (2006.01)
***C07K 14/08*** (2006.01)
***A61K 45/08*** (2006.01)
***A61P 31/16*** (2006.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 112778387
- CN 113307834


### Non-patent literature cited in the description


- **N. I. BRIKO ; A. YA. MINDLINA ; R. V. POLIBIN.** The universality of changes in the manifestations of the epidemic process of anthroponotic infections over the past decades. *Journal of Microbiology,* 2015, vol. 5, 12-20
- **M. L. AGOSTINI et al.** Small-molecule antiviral beta-d-N(4)-hydroxycytidine inhibits a proofreading-intact coronavirus with a high genetic barrier to resistance. *J. Virol.,* 2019, vol. 93, e01348-19
- **M. TOOTS et al.** Characterization of oral efficacious influenza drug with high resistance barrier in human airway epithelia. *Sci. Transl. Med,* 2019, vol. 11, eaax5866
- **F. KABINGER et al.** Mechanism of molnupiravir-induced SARS-CoV-2 mutagenesis. *Nature Structural & Molecular Biology,* 2021, vol. 28 (9), 740-746
- **N. URAKOVA et al.** β-d-N(4)-Hydroxycytidine Is a Potent Anti-alphavirus Compound That Induces a High Level of Mutations in the Viral Genome. *J. Virol.,* 2018, vol. 92
- **G. R. PAINTER et al.** The prophylactic and therapeutic activity of a broadly active ribonucleoside analog in a murine model of intranasal Venezuelan equine encephalitis virus infection. *Antiviral Res.,* 2019, vol. 171, 104597
- **O. REYNARD et al.** Identification of a new ribonucleoside inhibitor of Ebola virus replication. *Viruses,* 2015, vol. 7, 6233-6240
- **V. P. COSTANTINI et al.** Antiviral activity of nucleoside analogues against norovirus. *Antivir. Ther.,* 2012, vol. 17, 981-991
- **J. J. YOON et al.** Orally effective broad-spectrum ribonucleoside analog inhibitor of influenza And respiratory syncytial viruses. Antimicrob. *Agents Chemother.,* 2018, vol. 62, e00766-18
- **M. EHTESHAMI et al.** Characterization of beta-d-N(4)-hydroxycytidine as a novel inhibitor of Chikungunya virus. *Antimicrob. Agents Chemother.,* 2017, vol. 61, e02395-16
- **MART TOOTS et al.** Quantitative efficacy paradigms of the influenza clinical drug candidate EIDD-2801 in the ferret model. *Translational Research,* 2020, vol. 218, 16-28